(19) Europäisches Patentamt
European Patent Office
Office européen des brevets

(11) EP 0 601 303 B1

(12) EUROPÄISCHE PATENTSCHRIFT

(45) Veröffentlichungstag und Bekanntmachung des Hinweises auf die Patenterteilung:
**04.06.1997 Patentblatt 1997/23**

(51) Int Cl.[6]: **C07C 327/36**, C08K 5/39, C08L 9/00, C08J 3/24

(21) Anmeldenummer: **93116299.4**

(22) Anmeldetag: **08.10.1993**

(54) **Polysulfid-Verbindungen und Vulkanisationssysteme**

Polysulphide compounds and vulcanisation systems

Composés polysulfides et systèmes de vulcanisation

(84) Benannte Vertragsstaaten:
**AT BE CH DE ES FR GB IT LI LU NL SE**

(30) Priorität: **09.12.1992 DE 4241447**

(43) Veröffentlichungstag der Anmeldung:
**15.06.1994 Patentblatt 1994/24**

(73) Patentinhaber: **BAYER AG**
**51368 Leverkusen (DE)**

(72) Erfinder:
- **Wolpers, Jürgen, Dr.**
  **D-45721 Haltern (DE)**
- **Nordsiek, Karl-Heinz, Dr.**
  **D-45770 Marl (DE)**
- **Monkiewicz, Jaroslaw, Dr.**
  **D-45770 Marl (DE)**
- **Zerpner, Dieter, Dr.**
  **D-45770 Marl (DE)**

(56) Entgegenhaltungen:
**EP-A- 0 432 406**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist. (Art. 99(1) Europäisches Patentübereinkommen).

EP 0 601 303 B1

**Beschreibung**

Die Erfindung betrifft neue Polysulfid-Verbindungen aromatischer Dithiocarbonsäuren und ihre Verwendung als nitrosaminfreie Vernetzungsmittel und in Vulkanisationssystemen für Dien-Kautschuk zur Verbesserung der Reversions- und Alterungsstabilität.

Die Vernetzung von Kautschuk mit Schwefel oder Schwefelspendern und Beschleunigersystemen liefert in der Regel Vulkanisate, deren Polymerketten in der Anfangsphase durch polysulfidische Schwefelnetzbrücken verknüpft sind. Polysulfidisch vernetzte Vulkanisate führen bei einer Vielzahl von Eigenschaften zu guten Ergebnissen. Dies trifft neben der mechanischen Festigkeit insbesondere auf den Weiterreißwiderstand als auch auf den Abriebwiderstand zu. Ein Nachteil liegt jedoch in der Reversion solcher schwefelhaltigen Systeme. Unter dem Begriff Reversion faßt man den Wiederabfall der Vulkanisateigenschaften einerseits während des Vulkanisationsprozesses und andererseits die anaerobe Alterung bei dynamischer Beanspruchung zusammen. Der bei der Reversion freiwerdende Schwefel lagert sich einerseits an Polymersegmente an und erhöht damit deren Glastemperatur $T_g$ und katalysiert zum anderen den Angriff des Sauerstoffes auf das Vulkanisat. Beide Effekte führen zu einer Schädigung des Netzwerks. Die genannten Folgereaktionen des Schwefels, die in Abhängigkeit von Reaktionszeit und -temperatur bereits im Verlauf des Vulkanisationsprozesses einsetzen, verringern wesentlich die Leistungsgrenzen des Elastomeren und erfordern daher Beschränkungen im Hinblick auf die optimale Gestaltung des Fertigungsprozesses.

Eine Maßnahme zur Verringerung der Folgereaktionen des Schwefels stellt die Anwendung sogenannter EV-Systeme dar; dies sind Systeme, die aus erhöhten Mengen Vulkanisationsbeschleuniger bestehen bei gleichzeitig minimalen Mengen an Schwefel. Infolge der rascheren Anvulkanisation wird die Verarbeitungssicherheit jedoch nachteilig verändert. Dazu kommt - und dies ist von noch größerer Tragweite - die vorprogrammierte Beeinträchtigung des Abriebwiderstandes, der Weiterreißfestigkeit und der Kordhaftung gerade im Hinblick auf die Reifentechnologie (vgl. P. M. Lewis, NR Technologie 17, (4), 1986, S. 60).

In der DE-OS 39 41 002 (EP-A 0 432 406) werden u. a. Polysulfidderivate mit substituierten Hydroxydithiobenzoaten als Abgangsgruppen vorgeschlagen. Diese kompliziert aufgebauten Verbindungen haben gravierende Nachteile: Aufgrund des hohen Molgewichtes müssen, wie aus den Beispielen ersichtlich, 7 Teile Vernetzersubstanz auf 100 Teile Kautschuk eingesetzt werden, um Vulkanisate mit akzeptablem Eigenschaftsniveau zu erhalten. Dies belastet das Verfahren nicht nur wirtschaftlich, sondern es sind aufgrund der sehr begrenzten Löslichkeit solcher Verbindungen äußerst unerwünschte Ausblüherscheinungen zu befürchten.

Die mangelhafte Löslichkeit sowie der hohe Schmelzpunkt der in den Beispielen genannten Substanzen führt außerdem zu Schwierigkeiten beim Einmischen und, damit verbunden, zu mangelhafter Dispergierung der Substanz in der Kautschukmischung mit all seinen negativen Folgeerscheinungen.

Es bestand die Aufgabe, neue Vulkanisationsmittel sowie Vulkanisationssysteme zu finden, welche reversionsfreie und alterungsstabile Vulkanisate von Dien-Kautschuk liefern, die die Nachteile von EV-Systemen vermeiden und zugleich keine nitrosaminbildende Substanzen enthalten.

Die Aufgabe wurde durch Polysulfid-Verbindungen der allgemeinen Formel

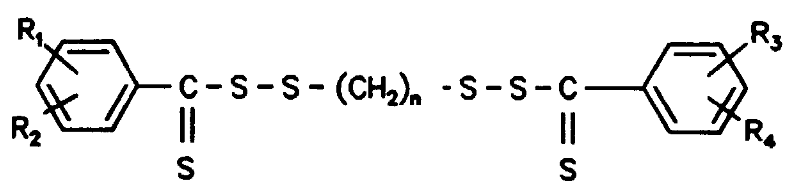

,

wobei $R_1$ - $R_4$ entweder Wasserstoff oder einen Alkylrest mit 1 - 4 C-Atomen bedeuten, die Substituenten $R_1$ - $R_4$ gleich oder verschieden sein können und n eine Zahl von 2 - 10 ist, gelöst.

Polysulfid-Verbindungen der obigen allgemeinen Formel sind niedrig-schmelzend und weisen eine gute Löslichkeit in Dien-Kautschukmischungen auf. Bei niedrigen Molgewichten der Reste $R_1$ - $R_4$ sowie einem niedrigen bis mittleren Wert für n zwischen 2 und 10 ist auch das Molgewicht des Vulkanisationsmittels niedrig genug, um eine deutlich verringerte vulkanisationswirksame Menge erforderlich zu machen.

Bevorzugte Substituenten $R_1$ - $R_4$ sind Wasserstoff oder eine Methylgruppe, wobei die Stellung der Methylgruppe ortho, meta oder para zur Thiocarbonyl-Gruppe sein kann. Die $CH_2$-Brücke kann zwischen 2 und 10 C-Atomen lang sein, wobei Verbindungen mit $C_2$- oder $C_6$-Ketten bevorzugt sind. Die Abgangsgruppe der Polysulfid-Verbindungen bei der Vulkanisation ist $C_6H_5CS_2^{\theta}$. Sie stellen je nach Gegenion eine flüssig bis niedrig schmelzende Substanz dar, die leicht in Kautschukmischungen einzuarbeiten ist.

Des weiteren wurde gefunden, daß man die erforderliche Menge der Polysulfid-Verbindungen bei Bedarf weiter

verringern kann, wenn man sie in speziellen Vulkanisationssystemen einsetzt. Diese Vulkanisationssysteme sind Mischungen aus Polysulfid-Verbindungen, die außerdem sehr geringe Mengen Schwefel sowie einen oder mehrere Zusatzbeschleuniger und ferner eine basische Substanz enthalten. Hierbei wird nicht etwa, wie zu erwarten, das Reversions- und Alterungsverhalten der Vulkanisate verschlechtert - also dem der üblichen Schwefelvulkanisate angenähert, sondern es ergibt sich überraschenderweise gerade der umgekehrte Effekt: Die Reversion wird vollständig unterdrückt, und die aerobe Alterungsbeständigkeit, also die Retention der physikalischen Eigenschaften nach Heißluftalterung wird deutlich verbessert. Durch diese nicht vorhersehbare Wirkung des von uns gefundenen Vulkanisationssystems werden Vulkanisate von Dien-Kautschuken mit sehr hoher Reversions- und Alterungsbeständigkeit erhalten.

Beispiele für die erfindungsgemäßen Polysulfid-Verbindungen sind insbesondere Bis-1,6-(thiobenzoyldisulfido)-ethan und -hexan, hiernach BTBDE und BTBDH genannt, die auch bevorzugt zur Anwendung kommen. Die Polysulfid-Verbindungen können als Vernetzersubstanzen in reiner Form, als konzentrierte Mischungen in Kautschuken (sogenannte Batche) oder als Mischungen mit Füllstoffen eingesetzt werden.

Werden die Polysulfid-Verbindungen allein zur Vulkanisation eingesetzt, reichen zur Erzielung der erfindungsgemäßen Wirkung 4 bis 6 Teile aus. So erhält man zum Beispiele aus 5,7 Teilen der Verbindung mit $R_1$ - $R_4$ = H und n = 6 (BTBDH) auf 100 Teile Kautschuk ein Vulkanisat mit einem Spannungswert von 6,8 MPa bei 300 % Dehnung.

Deutlich geringere Mengen der Polysulfid-Verbindungen reichen für eine übliche Vernetzung aus, wenn man jeweils bezogen auf 100 Teile Kautschuk neben nur 0,5 bis 4 Teilen BTBDH oder BTBDE eine Menge von 0 bis 0,3 Teile Schwefel, 1 bis 2,5 Teile, bevorzugt 1 - 1,5 Teile, eines Zusatzbeschleunigers, z. B. ein Sulfenamid- oder ein Merkapto-Beschleuniger oder eine Kombination hiervon, sowie 0,5 bis 4 Teile, bevorzugt 0,5 bis 1,5 Teile, einer basischen Verbindung zusetzt. Durch diese Zusätze wird die Reversionsneigung von Dien-Kautschuken überraschend völlig unterdrückt und die aerobe Alterung deutlich verbessert.

Als bevorzugte Beschleuniger finden Zink-2-merkaptobenzothiazol (ZMBT), Dibenzothiazyldisulfid (MBTS) und N-tert-Butyl-2-benzothiazylsulfenamid (TBBS) Verwendung, andere Merkapto- oder Sulfenamidbeschleuniger sind jedoch durchaus im Rahmen der Erfindung anwendbar.

Erfindungsgemäß werden die Polysulfid-Verbindungen außer ohne auch mit Schwefel in Mengen bis 0,3 Teile pro 100 Teile Kautschuk zur Vulkanisation eingesetzt. Er bewirkt eine Erhöhung der Vernetzungsausbeute ohne Beeinträchtigung der Reversion oder des Alterungsverhaltens, wenn man im Rahmen der vorgegebenen Mengen bleibt. Bei höheren Mengen Schwefel als 0,3 Teile werden jedoch zunehmend Reversion und ungünstige Alterungseigenschaften beobachtet.

Die basischen Substanzen unterdrücken die Reversion, verbessern die Alterung und erhöhen die Vernetzungsausbeute. Insbesondere kommen Guanidinderivate in Frage, z. B. das Diphenylguanidin. Ferner sind auch Salze von Dithiocarbaminsäuren geeignet, wobei man jedoch darauf achten muß, Carbamate von sogenannten "Safe Amines" zu verwenden, die keine Nitrosamine entwickeln. Als repräsentatives Beispiel hierfür dient das Zink-Salz der Dibenzyldithiocarbaminsäure.

Es können weitere übliche Kautschukzusätze, wie z. B. Füllstoffe, Weichmacher, Klebrigmacher, Beschleuniger, Aktivatoren, Stearinsäure, Wachs, Alterungs- und Ozonschutzmittel, Treibmittel, Farbstoffe sowie Pigmente verwendet werden. Die Herstellung von Vulkanisationssystemen mit den genannten Kautschukzusätzen erfolgt nach üblichen, dem Fachmann bekannten Verfahren.

Die Vulkanisation wird bei Temperaturen zwischen 130 und 250 °C, bevorzugt zwischen 150 und 220 °C, durchgeführt. Die Vulkanisation geschieht ebenfalls nach üblichen, dem Fachmann bekannten Verfahren.

Die erfindungsgemäßen Polysulfidderivate weisen in den zu verwendenden Rezepturen Vorteile hinsichtlich Reversionsstabilität nach längerer Vulkanisationszeit und/oder höherer Vulkanisationstemperatur auf. Gleichzeitig zeigen sie in der aeroben Alterung der Elastomeren einen erheblich geringeren Leistungsabfall.

Die Herstellung der Polysulfidderivate und die Vorteile von Dien-Kautschukvulkanisaten durch deren Verwendung als Vernetzersubstanz allein und in Vulkanisationssystemen werden durch die nachfolgenden Beispiele erläutert.

1. Natriumdithiobenzoat

$$PhCCl_3 + 2\ Na_2S \times 2{,}7\ H_2O \rightarrow PhCS_2Na + 3\ NaCl\ (Ph = Phenyl)$$

415 g Natriumsulfidhydrat wurden in 1 000 ml Methanol suspendiert und auf Rückflußtemperatur erwärmt. Danach wurden 330.4 g Benzotrichlorid unter Rückfluß zugetropft und man läßt die Reaktionsmischung noch einige Minuten bei Siedetemperatur nachreagieren. Anschließend wurde das ausgefallene NaCl bei Raumtemperatur abgetrennt. Das Methanol wurde bei 50 °C abgedampft und der verbleibende Rückstand in ca. 1 900 ml destilliertem Wasser gelöst. Diese wäßrige Phase (2 200 ml) wurde zwecks Reinigung mit 500 ml Toluol extrahiert und die abgetrennte Toluolphase verworfen.

2. 1,6-Bis(natriumthiosulfato)hexan (BBS)

$$Cl(CH_2)_6Cl + 2\ Na_2S_2O_3 \times 5H_2O \rightarrow NaO_3S_2(CH_2)_6S_2O_3Na + 2\ NaCl$$

Zu einer Lösung von 374.5 g Natriumthiosulfatpentahydrat in ca. 1 150 ml Wasser wurden bei Raumtemperatur 108.5 g 1,6-Dichlorhexan zugegeben. Die Reaktionsmischung wurde danach durch Zutropfen von 5%iger NaOH auf ph-Wert 7 bis 8 eingestellt. Gleichzeitig wurde der gesamte Kolbeninhalt auf Rückflußtemperatur erwärmt und 7 - 8 Stunden bei der Temperatur gerührt. Anschließend wurde die Mischung auf Raumtemperatur gekühlt und mit 150 ml Methyltertiärbutylether (MTB) extrahiert. Der MTB-Extrakt wurde verworfen und die wäßrige Bisbuntesalzlösung (BBS) (ca. 1 500 ml) in der nächsten Stufe eingesetzt.

3. 1,6-Bis(thiobenzoyldisulfido)hexan

$$2\ PhCS_2Na + NaO_3S_2(CH_2)_6S_2O_3Na \text{ -----> } Ph\overset{S}{\overset{\|}{C}}S_2(CH_2)_6S_2\overset{S}{\overset{\|}{C}}Ph + 2\ Na_2SO_3$$

In einen 5 l-Kolben mit Rührer, Tropftrichter und pH-Meßelektrode wurden die einzelnen Reaktionskomponenten in folgender Reihenfolge bei Raumtemperatur zugegeben:

a) 1 500 ml Bisbuntesalzlösung (Punkt 2)
b) 100 g 37%ige Formalinlösung
c) 2 200 ml Natriumdithiobenzoatlösung (Punkt 1).

Unter sehr starkem Rühren wurde der pH-Wert durch Zutropfen von 10%iger HCl auf pH 8 eingestellt. Die Reaktionsmischung wurde 7 h bei Raumtemperatur und pH 7 - 8 kräftig gerührt. Es entstand ein dunkelrotes Öl, das nach Reaktionsende von der wäßrigen Phase abgetrennt wurde. Die Ölphase wurde mit 1 500 ml Toluol verdünnt und 3 Mal mit ca. 700 ml $H_2O$ gewaschen. Die organische Phase wurde über Magnesiumsulfat (Natriumsulfat) getrocknet. Nach Abnutschen des Trocknungsmittels wurde das Toluol unter Vakuum abgedampft. Es blieben 220 g des Produkts in Form eines dunkelroten zähflüssigen Öls, Ausbeute 70 %.

Beispiel 1 demonstriert die Verwendung von BTBDH in einer Naturkautschuk-Rezeptur. Gegenüber Vergleichsbeispiel I, der gleichen Naturkautschuk-Grundmischung mit einem herkömmlichen Vulkanisationssystem aus zwei Teilen Schwefel und einem Teil des Sulfenamidbeschleunigers CBS zeigen sich

a) die Vernetzungsaktivität der erfindungsgemäßen Substanz,
b) die überraschend kurzen Anvulkanisations- und Ausvulkanisationszeiten selbst bei 150 °C sowie
c) die deutlich verbesserte Alterungsstabilität der Rezeptur gegenüber dem Vergleichsbeispiel.

Beispiel 2 demonstriert den Einsatz von 3,5 Teilen BTBDH sowie zusätzlich 1,5 Teilen MBTS als Zusatzbeschleuniger und ferner 1,5 Teilen ZBEC als basischer Verbindung - jedoch ohne Schwefel - in einer cis-Polyisopren-Rezeptur. Mit deutlich reduzierter Menge des erfindungsgemäßen Vulkanisationsmittels ist dennoch ein höherer Spannungswert des Vulkanisates erzielt worden als in Beispiel 1. Dies ist überraschend, da die zusätzlich verwendeten Substanzen MBTS und ZBEC für sich allein keinerlei Vulkanisationsaktivität entfalten. Vergleiche hierzu auch das Vergleichsbeispiel II, das totz einer wesentlich höheren Menge Schwefel nur einen unzureichenden Spannungswert liefert.

Beispiel 3 demonstriert die Verwendung von nur 2 Teilen BTBDH in der gleichen Grundmischung wie in Beispiel 2, jedoch mit 0,2 Teile Schwefel und 1,5 Teilen MBTS als Zusatzbeschleuniger sowie ein Teil ZBEC als basischer Substanz. In dieser Zusammensetzung ist eine Retention von 72 % der ursprünglichen Reißdehnung des Vulkanisates nach 14tägiger Alterung bei 100 °C zu beobachten.

Beispiel 4 zeigt die Verwendung von Diphenylguanidin (DPG) als basische Substanz neben TBBS als Zusatzbeschleuniger. Dabei erzielt man ein Vulkanisat mit sehr hoher dynamischer Beständigkeit, die sich auch im Verlaufe einer 14tägigen Alterung bei 100 °C nicht verschlechtert. Die Retention der Reißdehnung ist 73 %.

Beispiel 5 zeigt den Einsatz von nur 0,5 Teile BTBDH zusammen mit 0,3 Teile Schwefel, 2 Teilen TBBS als Zusatzbeschleuniger und 1,5 Teilen ZBEC als basiche Substanz. Es wird ein Vulkanisat mit einem Modul (Spannungswert bei 300 % Dehnung) von 0,8 MPa erreicht. Nach 14tägiger Alterung bei 100 °C weist das Vulkanisat 71 % Retention bei der ReiBdehnung auf.

Die verschiedenen Ausführungsformen des erfindungsgemäßen Verfahrens, die obenstehend nur anhand einiger Beispiele wiedergegeben wurden, belegen die erzielbaren außergewöhnlichen Eigenschaften der Vulkanisate selbst bei einem so empfindlichen und unter normalen Bedingungen wenig alterungsstabilen Kautschuk wie dem cis-Polyisopren.

Erläuterungen zu den Tabellen:

1) RSS # 1 Defo 1000
2) NATSYN®, Goodyear
3) N-Isopropyl-N'-phenyl-p-phenylendiamin
4) N-(1,3-dimethylbutyl)-N'-phenyl-p-phenylendiamin
5) N-Cyclohexyl-1-benzothiazylsulfenamid
6) N-tert-Butyl-2-benzothiazylsulfenamid (TBBS)
7) Dibenzothiazyldisulfid (MBTS)
8) Diphenylguanidin (DPG)
9) Zink-dibenzyldithiocarbamt
10) Bis-1,6-(thiobenzoyldisulfido)-hexan

': Zeit in Minuten, nach der die Kugel unter den angegebenen Bedingungen zerstört wurde.

Tabelle 1:

| Beispiel | | 1 | 2 | 3 | 4 | 5 |
|---|---|---|---|---|---|---|
| Naturkautschuk [1)] | | 100,0 | - | - | - | - |
| cis-Polyisopren [2)] | | - | 100,0 | 100,0 | 100,0 | 100,0 |
| ZnO RS | | 3,0 | 3,0 | 3,0 | 3,0 | 3,0 |
| Stearinsäure | | 2,0 | 2,0 | 2,0 | 2,0 | 2,0 |
| IPPD [3)] | | 1,0 | 1,0 | 1,0 | 1,0 | 1,0 |
| 6PPD [4)] | | 1,0 | 1,0 | 1,0 | 1,0 | 1,0 |
| Corax N-339 | | 45,0 | 45,0 | 45,0 | 45,0 | 45,0 |
| CBS [5)] | | - | - | - | - | - |
| Schwefel | | - | - | 0,2 | 0,3 | 0,3 |
| TBBS [6)] | | - | - | - | 1,5 | 2,0 |
| MBTS [7)] | | - | 1,5 | 1,5 | - | - |
| DPG [8)] | | - | - | - | 3,0 | - |
| ZBEC [9)] | | - | 1,5 | 1,0 | - | 1,5 |
| BTBDH [10)] | | 5,7 | 3,5 | 2,0 | 3,0 | 0,5 |
| Vulkameter 150 °C $t_{10}$ | min. | 1,9 | 1,3 | 1,5 | 1,0 | 2,5 |
| $t_{90}$ | min. | 4,5 | 14,0 | 11,5 | 8,6 | 11,2 |
| Vulkameter 180 °C $t_{10}$ | min. | 0,8 | 0,6 | 0,7 | 0,5 | 1,0 |
| $t_{90}$ | min. | 1,5 | 5,5 | 5,8 | 3,0 | 2,5 |
| Vulkanisation bei | °C/min. | 150/30 | 180/10 | 180/10 | 180/10 | 180/10 |
| Zugfestigkeit | MPa | 18,0 | 21,1 | 30,3 | 20,7 | 21,1 |
| Bruchdehnung | % | 583 | 589 | 579 | 572 | 537 |
| Modul 100 % Dehnung | MPa | 1,4 | 1,4 | 1,4 | 1,4 | 1,6 |
| Modul 300 % Dehnung | MPa | 7,4 | 7,9 | 8,0 | 8,1 | 9,8 |
| Struktur n. Pohle | N/mm | 66 | 66 | 68 | 74 | 65 |
| bleibende Dehnung | % | 16 | 9 | 8 | 8 | 8 |

Fortsetzung Tabelle 1:

| Beispiel | | | 1 | 2 | 3 | 4 | 5 |
|---|---|---|---|---|---|---|---|
| Härte | 22 °C | Shore A | - | 56 | 56 | 58 | 57 |
| | 75 °C | | - | 48 | 49 | 48 | 48 |
| Elastizität | 22 °C | | - | 51 | 51 | 50 | 48 |
| | 75 °C | | - | 65 | 61 | 64 | - |
| Compr. set 24h/70 °C | | % | - | 9 | 11 | 11 | 11 |
| Compr. set 24h/100°C | | % | - | 27 | 27 | 28 | 34 |
| Vulkanisation bei 180 °C min. | | | - | 20 | 20 | 20 | 20 |
| Kugelzermürbung | 150N | °C | - | 86 | 91 | 82 | 86 |
| | 200N | °C | - | 110 | 120 | 103 | 110 |
| | 250N | °C | - | 124 | 144 | 120 | 132 |
| | 300N | °C | - | 139 | 167 | 133 | 145 |
| | 350N | °C | - | 160 | 6' | 152 | 168 |
| | 400N | °C | - | 8' | - | 178 | 5' |
| | 450N | °C | - | - | - | 3' | - |

Alterung 7 Tage bei 100 °C

| Beispiel | | 1 | 2 | 3 | 4 | 5 |
|---|---|---|---|---|---|---|
| Zugfestigkeit | MPa | 7,0 | 20,4 | 20,0 | 17,7 | 19,5 |
| Bruchdehnung | % | 289 | 428 | 483 | 521 | 443 |
| Modul 100 % Dehnung | MPa | 2,0 | 2,8 | 2,2 | 1,8 | 2,4 |
| Modul 300 % Dehnung | MPa | - | 14,2 | 11,5 | 9,2 | 12,8 |
| Struktur n. Pohle | N/mm | 17 | 56 | 55 | 36 | 57 |
| bleibende Dehnung | % | 8 | 9 | 9 | 14 | 9 |

Fortsetzung Tabelle 1:

| | | | Alterung 7 Tage bei 100 °C | | | | |
|---|---|---|---|---|---|---|---|
| Beispiel | | | 1 | 2 | 3 | 4 | 5 |
| Härte | 22 °C | Shore A | - | 63 | 62 | 64 | 63 |
| | 75 °C | | - | 55 | 54 | 58 | 56 |
| Elastizität | 22 °C | | - | 54 | 52 | 56 | 52 |
| | 75 °C | | - | 62 | 61 | 65 | 61 |
| Compr. set 24h/ 70 °C | | % | - | 17 | 16 | 12 | 15 |
| Compr. set 24h/100 °C | | % | - | 26 | 33 | 24 | 34 |
| Kugelzermürbung | 150N | °C | - | 84 | 75 | 78 | 86 |
| | 200N | °C | - | 111 | 108 | 94 | 103 |
| | 250N | °C | - | 134 | 132 | 114 | 132 |
| | 300N | °C | - | 141 | 157 | 134 | 150 |
| | 350N | °C | - | 156 | 164 | 150 | 170 |
| | 400N | °C | - | 14,8' | 14,8' | 169 | 5' |
| | 450N | °C | - | - | - | 12' | - |

| | | Alterung 14 Tage bei 100 °C | | | | |
|---|---|---|---|---|---|---|
| Beispiel | | 1 | 2 | 3 | 4 | 5 |
| Zugfestigkeit | MPa | 5,7 | 15,8 | 17,3 | 11,8 | 16,1 |
| Bruchdehnung | % | 240 | 331 | 415 | 417 | 380 |
| Modul 100 % Dehnung | MPa | 2,3 | 3,2 | 2,5 | 1,9 | 2,6 |
| Modul 300 % Dehnung | MPa | - | 15,7 | 12,7 | 8,5 | 13,1 |
| Struktur n. Pohle | N/mm | 14 | 37 | 41 | 18 | 35 |
| bleibende Dehnung | % | 8 | 7 | 11 | 14 | 9 |

Fortsetzung Tabelle 1:

| Beispiel | | | Alterung 14 Tage bei 100 °C 1 | 2 | 3 | 4 | 5 |
|---|---|---|---|---|---|---|---|
| Härte | 22 °C | Shore A | - | 62 | 64 | 64 | 65 |
| | 75 °C | | - | 52 | 55 | 55 | 55 |
| Elastizität | 22 °C | | - | 51 | 50 | 52 | 50 |
| | 75 °C | | - | 59 | 59 | 60 | 58 |
| Compr. set 24h/ 70 °C | | % | - | 23 | 26 | 21 | 20 |
| Compr. set 24h/100 °C | | % | - | 34 | 39 | 29 | 39 |
| Kugelzermürbung | 150N | °C | - | 83 | 74 | 72 | 85 |
| | 200N | °C | - | 109 | 102 | 92 | 101 |
| | 250N | °C | - | 130 | 128 | 110 | 128 |
| | 300N | °C | - | 147 | 154 | 122 | 153 |
| | 350N | °C | - | 14' | 9' | 144 | 13' |
| | 400N | °C | - | - | - | 158 | - |
| | 450N | °C | - | - | - | 11' | - |

Tabelle 2:

| Vergleichsbeispiel | | I | II |
|---|---|---|---|
| Naturkautschuk (1) | | 100,0 | 100,0 |
| cis-Polyisopren (2) | | - | - |
| ZnO RS | | 3,0 | 3,0 |
| Stearinsäure | | 2,0 | 2,0 |
| Vulk. 4010 NA (3) | | 1,0 | 1,0 |
| Vulk. 4020 (4) | | 1,0 | 1,0 |
| Corax N-339 | | 45,0 | 45,0 |
| CBS (5) | | 1,0 | - |
| Schwefel | | 2,0 | 0,4 |
| TBBS (6) | | - | 1,2 |
| MBTS (7) | | - | - |
| DPG (8) | | - | - |
| ZBEC (9) | | - | - |
| BTBDH | | - | - |
| Vulkameter 150 °C $t_{10}$ | min. | 5,0 | 8,4 |
| $t_{90}$ | min. | 9,2 | 14,4 |
| Vulkameter 180 °C $t_{10}$ | min. | 1,4 | - |
| $t_{90}$ | min. | 2,3 | - |
| Vulkanisation bei | °C min. | 150/30 | 150/30 |
| Zugfestigkeit | MPa | 19,3 | 19,5 |
| Bruchdehnung | % | 480 | 528 |
| Modul 100 % Dehnung | MPa | 1,6 | 1,5 |
| Modul 300 % Dehnung | MPa | 10,5 | 9,2 |
| Struktur n. Pohle | N/mm | 37 | 66 |
| bleibende Dehnung | % | 10 | 12 |

Fortsetzung Tabelle 2:

| Vergleichsbeispiel | | | I | II |
|---|---|---|---|---|
| Härte | 22 °C | Shore A | - | 55 |
| | 75 °C | | - | 44 |
| Elastizität | 22 °C | | - | 50 |
| | 75 °C | | - | 58 |
| Compr. set 24h/ 70 °C | | % | - | 36 |
| Compr. set 24h/100 °C | | % | - | - |
| Vulkanisation bei 180°C | | min. | - | - |
| Kugelzermürbung | 150N | °C | - | - |
| | 200N | °C | - | - |
| | 250N | °C | - | - |
| | 300N | °C | - | - |
| | 350N | °C | - | - |
| | 400N | °C | - | - |
| | 450N | °C | - | - |

| | | | Alterung 7 Tage bei 100 °C | |
|---|---|---|---|---|
| Vergleichsbeispiel | | | I | II |
| Zugfestigkeit | | MPa | 4,5 | 11,1 |
| Bruchdehnung | | % | 105 | 391 |
| Modul 100 % Dehnung | | MPa | - | 1,9 |
| Modul 300 % Dehnung | | MPa | 16,0 | 8,9 |
| Struktur n. Pohle | | N/mm | 1 | 13 |
| bleibende Dehnung | | % | - | 11 |
| Härte | 22 °C | Shore A | - | 61 |
| | 75 °C | | - | 49 |

Fortsetzung Tabelle 2:

| Vergleichsbeispiel | | Alterung 7 Tage bei 100 °C I | II |
|---|---|---|---|
| Elastizität 22 °C | | - | 46 |
| 75 °C | | - | 50 |
| Compr. set 24h/ 70 °C | % | - | 28 |
| Compr. set 24h/100 °C | % | - | - |
| Kugelzermürbung 150N | °C | - | - |
| 200N | °C | - | - |
| 250N | °C | - | - |
| 300N | °C | - | - |
| 350N | °C | - | - |
| 400N | °C | - | - |
| 450N | °C | - | - |

| Vergleichsbeispiel | | Alterung 14 Tage bei 100 °C I | II |
|---|---|---|---|
| Zugfestigkeit | MPa | 3,0 | 66,0 |
| Bruchdehnung | % | 54 | 311 |
| Modul 100 % Dehnung | MPa | - | 1,7 |
| Modul 300 % Dehnung | MPa | - | 6,5 |
| Struktur n. Pohle | N/mm | 18 | 12 |
| bleibende Dehnung | % | 1 | 10 |
| Härte 22 °C | Shore A | - | 60 |
| 75 °C | | - | 47 |
| Elastizität 22 °C | | - | 42 |
| 75 °C | | - | 45 |

Fortsetzung Tabelle 2:

| Vergleichsbeispiel | | Alterung 14 Tage bei 100 °C I | II |
|---|---|---|---|
| Compr. set 24h/ 70 °C | % | - | 40 |
| Compr. set 24h/100 °C | % | - | - |
| Kugelzermürbung 150N | °C | - | - |
| 200N | °C | - | - |
| 250N | °C | - | - |
| 300N | °C | - | - |
| 350N | °C | - | - |
| 400N | °C | - | - |
| 450N | °C | - | - |

**Patentansprüche**

1. Polysulfid-Verbindungen der allgemeinen Formel

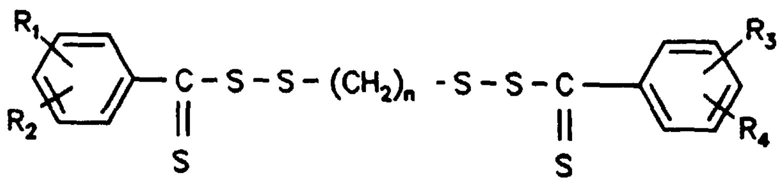

,

wobei $R_1$ - $R_4$ gleichzeitig oder unabhängig voneinander Wasserstoff oder einen Alkylrest mit 1 bis 4 Kohlenstoffatomen bedeuten und n gleich 2 bis 10 ist.

2. Polysulfid-Verbindungen nach Anspruch 1, dadurch gekennzeichnet, daß $R_1$ - $R_4$ gleich Wasserstoff und n = 2 oder 6 bedeuten.

3. Verwendung von 0,5 bis 6,0 Teile Polysulfid-Verbindung nach den Ansprüchen 1 - 2 zur Vulkanisation von Dien-Kautschuk.

4. Vulkanisationssysteme im wesentlichen enthaltend

   a) 0,5 - 6 Teile Polysulfid-Verbindung nach den Ansprüchen 1 - 2,
   b) 0 - 0,3 Teile Schwefel,
   c) 1 - 2,5 Teile Zusatzbeschleuniger,
   d) 0,5 - 4 Teile basische Verbindungen und
   e) Kautschukzusätze.

5. Vulkanisationssysteme nach Anspruch 4, dadurch gekennzeichnet, daß als Zusatzbeschleuniger Sulfenamid- und/oder Merkaptobeschleuniger verwendet werden.

6. Vulkanisationssysteme nach den Ansprüchen 4 - 5, dadurch gekennzeichnet, daß als Merkaptobeschleuniger Zink-2-merkaptobenzothiazol und/oder Dibenzothiazyldisulfid und als Sulfenamidbeschleuniger N-tert-Butyl-2-benzothiazylsulfenamid verwendet werden.

7. Vulkanisationssysteme nach den Ansprüchen 4 - 6, dadurch gekennzeichnet, daß als basische Verbindungen Guanidin-Verbindungen und/oder Salze einer Dithiocarbaminsäure verwendet werden.

8. Verfahren zur Herstellung von Dienkautschukvulkanisaten in Gegenwart von üblichen Kautschukzusätzen, dadurch gekennzeichnet, daß 0,5 - 6 Teile Polysulfid-Verbindungen der allgemeinen Formel

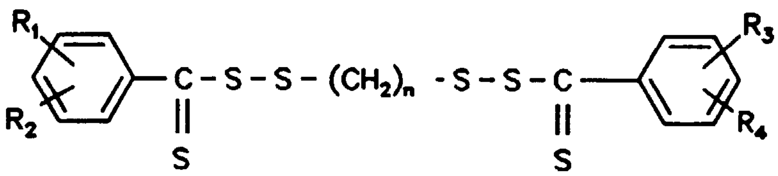

verwendet werden, wobei $R_1$ - $R_4$ gleichzeitig oder unabhängig voneinander Wasserstoff oder einen Alkylrest mit 1 bis 4 Kohlenstoffatomen bedeuten und n gleich 2 bis 10 ist, bei Temperaturen von 130 bis 250 °C.

9. Verfahren nach Anspruch 8, dadurch gekennzeichnet, daß $R_1$ - $R_4$ gleich Wasserstoff und n = 2 oder 6 bedeuten.

10. Verfahren zur Herstellung von Dien-Kautschukvulkanisaten im wesentlichen enthalten Vulkanisationssysteme nach den Ansprüchen 4 bis 7 bei Temperaturen von 130 bis 200 °C.

**Claims**

1. A polysulphide compound of the general formula

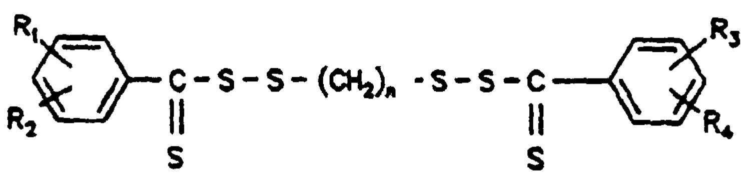

,

in which $R_1$ - $R_4$, simultaneously or independently of one another, denote hydrogen or an alkyl radical having 1 to 4 carbon atoms and n is 2 to 10.

2. A polysulphide compound according to claim 1, characterized in that $R_1$ - $R_4$ denote hydrogen and n denotes 2 or 6.

3. Use of 0.5 to 6.0 parts of a polysulphide compound according to either of claims 1 - 2 for the vulcanization of diene rubber.

4. A vulcanization system essentially contains

a) 0.5 - 6 parts of a polysulphide compound according to either of claims 1 - 2,
b) 0 - 0.3 part of sulphur,
c) 1 - 2.5 parts of additional accelerators,
d) 0.5 - 4 parts of basic compounds and

e) rubber additives.

5. A vulcanization system according to claim 4, characterized in that sulphenamide and/or mercapto accelerators are used as additional accelerators.

6. A vulcanization system according to either of claims 4 - 5, characterized in that zinc 2-mercaptobenzothiazole and/or dibenzothiazyl disulphide are used as mercapto accelerators and N-tert-butyl-2-benzothiazylsulphenamide is used as a sulphenamide accelerator.

7. A vulcanization system according to any of claims 4 - 6, characterized in that guanidine compounds and/or salts of a dithiocarbamic acid are used as basic compounds.

8. A process for the preparation of vulcanized diene rubbers in the presence of conventional rubber additives, characterized in that 0.5 - 6 parts of polysulphide compounds of the general formula

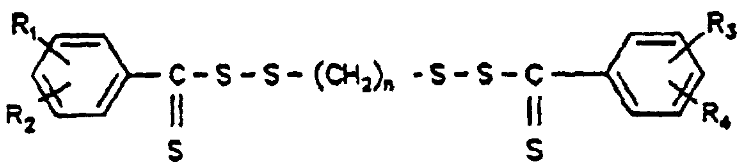

in which $R_1$ - $R_4$, simultaneously or independently of one another, denote hydrogen or an alkyl radical of 1 to 4 carbon atoms and n is 2 to 10, are used at temperatures of from 130 to 250°C.

9. A process according to claim 8, characterized in that $R_1$ - $R_4$ denote hydrogen and n denotes 2 or 6.

10. A process for the preparation of vulcanized diene rubbers essentially containing vulcanization systems according to any of claims 4 to 7 at temperatures of from 130 to 200°C.

**Revendications**

1. Composés polysulfurés de formule générale :

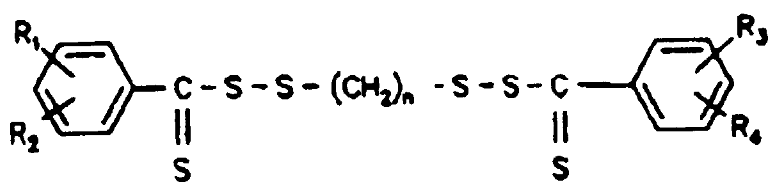

dans laquelle $R_1$ à $R_4$, simultanément ou indépendamment l'un de l'autre, signifient de l'hydrogène ou un radical alkyle ayant de 1 à 4 atomes de carbone et n est égal à 2 à 10.

2. Composés polysulfurés selon la revendication 1,
caractérisés en ce que
$R_1$-$R_4$ signifient de l'hydrogène et n égal à 2 ou 6.

3. Utilisation de 0,5 à 6,0 parties de composé polysulfuré selon les revendications 1-2 pour la vulcanisation du caoutchouc-diène.

4. Procédés de vulcanisation contenant essentiellement :

a) 0,5-6 parties de composé polysulfuré selon les revendications 1 et 2,
b) 0-0,3 partie de soufre,

c) 1-2,5 parties d'accélérateur d'addition,
d) 0,5-4 parties de composés basiques et,
e) des additifs de caoutchouc.

**5.** Procédés de vulcanisation selon la revendication 4, caractérisés en ce que l'on utilise comme accélérateur d'addition un accélérateur sulfénamidé et/ou un accélérateur mercapto.

**6.** Procédés de vulcanisation selon les revendications 4 et 5, caractérisés en ce que l'on utilise comme accélérateur mercapto, du 2-mercaptobenzothiazole zincique et/ou du disulfure de dibenzoylthiazyle et comme accélérateur sulfénamidique le N-ter-butyl-2-benzothiazylculfénamide.

**7.** Procédés de vulcanisation selon les revendications 4-6, caractérisés en ce que l'on utilise comme composés basiques des composés guanidiniques et/ou des sels d'un acide dithiocarbamique.

**8.** Procédé de fabrication de vulcanisats de caoutchouc-diène en présence d'additifs usuels de caoutchouc, caractérisé en ce que l'on utilise 0,5 à 6 parties de composés polysulfurés de formule générale :

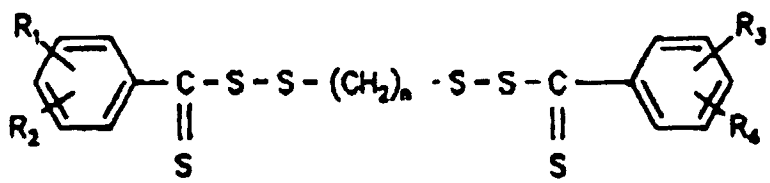

dans laquelle $R_1$-$R_4$, simultanément ou indépendamment l'un de l'autre, signifient de l'hydrogène ou un radical alkyle ayant de 1 à 4 atomes de carbone,
et n est égal de 2 à 10
à des températures de 130 à 250°C.

**9.** Procédé selon la revendication 8, caractérisé en ce que $R_1$-$R_4$ signifient un hydrogène identique et n = 2 ou 6.

**10.** Procédé de fabrication de vulcanisats de caoutchouc-diène renfermant essentiellement des procédés de vulcanisation selon les revendications 4 à 7, à des températures allant de 130 à 200°C.